# EUROPEAN PATENT SPECIFICATION

(11) **EP 4 456 937 B1**
(45) Date of publication and mention of the grant of the patent: **26.08.2026**
(21) Application number: 22830407.7
(22) Date of filing: 01.12.2022
(51) Int. Cl.: A61L 27/36

(54) **BONE GRAFT SUBSTITUTES**
ERSATZ FÜR KNOCHENTRANSPLANTATE
SUBSTITUTS DE GREFFON OSSEUX

(30) Priority: 02.12.2021 GB 202117457
(43) Date of publication of application: 06.11.2024
(73) Proprietor: ZOAN NUAIL TEORANTA, H91VW58 Co Galway (IE)
(72) Inventor: JOHNSON, Martin, Cloonacarton, Recess, H91 VW58 (IE); MARTIN, James, Cloonacarton, Recess, H91 VW58 (IE); WANN, Stephen, Cloonacarton, Recess, H91 VW58 (IE)
(74) Representative: Abel & Imray LLP
(86) International application number: PCT/EP2022/084141
(87) International publication number: WO 2023/099702

(56) References cited:
- US-A1- 2013 226 310
- FANTAZZINI PAOLA ET AL: "Gains and losses of coral skeletal porosity changes with ocean acidification acclimation", NATURE COMMUNICATIONS, vol. 6, no. 1, 1 November 2015 (2015-11-01), XP055838808, Retrieved from the Internet <URL:https://www.nature.com/articles/ncomms8785.pdf> DOI: 10.1038/ncomms8785

## Description

### BACKGROUND

The present disclosure relates to a method for manufacturing a coral scaffold for use as a bone graft substitute. The present invention also relates to a bone graft substitute.

Bone graft substitutes are porous materials that provide an osteoconductive scaffold for the growth of bone-forming tissues. Various synthetic bone graft substitutes are known. Examples include ceramic products based on calcium phosphate, such as hydroxyapatite and tricalcium phosphate. Ceramic bone graft substitutes are commercially available under the trademark ProOsteon^{®}. Natural coral has also been described for use as a bone graft substitute. US 2013/226310 A1 discloses a method of producing bioactive coral bone graft substitutes by enriching the coral growth medium with a nutrient (silicate or phosphate) to stimulate growth and alter the composition of the coral tissue.

### BRIEF DESCRIPTION OF FIGURES

Figure 1 shows an example of the pH response of a growth medium (e.g. seawater) to the addition of 4mg/L of NaOH at a range of dKH and initial pH values;
Figure 2 presents photo-microscopy images of corals from Example 1, grown under normal (dKH around 8.5) and low dKH (below 6.5) conditions;
Figure 3 shows the pH - solubility relationship for certain metal carbonates in accordance with Example 3;
Figure 4 shows CaCO₃ (aragonite) saturation state over a range of dKH and pH values in a growth medium with the composition of standard seawater at 25 degrees celcius with a) calcium ion concentration of 480ppm and b) calcium ion concentration of 640ppm in accordance with Example 4; and
Figure 5 presents photo-microscopy images of corals grown at low carbonate availability but high pH (achieved by addition of sodium hydroxide solution to the growth medium).

### DESCRIPTION

The present disclosure relates to a method of manufacturing a coral scaffold for use as a bone graft substitute. The method comprises of growing coral in a growth medium, removing at least a portion of the coral from the growth medium, and devitalising coral removed from the growth medium. The pH of the growth medium is controlled at a pH of 8.3 or greater, and carbonate hardness, dKH, is controlled at less than 10. The devitalised coral may be sized and shaped to form a bone graft substitute.

In some examples, the method comprises independently controlling the pH and dKH of the growth medium.

The pH and/or dKH may be controlled by e.g. maintaining or modifying these parameters in the target ranges.

Carbonate hardness, dKH, is a measure of the growth medium's ability to resist changes in pH. It is determined by the amount of bicarbonate and carbonate ions in the water. A growth medium having a low dKH has a reduced ability to resist changes in pH than a growth medium having a higher dKH. dKH, is analogous to "alkalinity". However, dKH is not the same as pH; the latter is a measure of the acidity or basicity (specifically the H⁺ concentration).

dKH as referred to herein can be defined as the sum of carbonate (CO₃²) and bicarbonate (HCO₃⁻) ions in solution, where 1 dKH = 17.9 ppm total of carbonate plus bicarbonate ions. In some cases, dKH may be measured using a pH-based titration which measures so-called 'titration alkalinity' which measures the all species contributing to alkalinity, not only carbonate ions. However, in some embodiments of the present disclosure, the pH of the growth medium may be controlled using non-carbonate sources of alkalinity. In order to measure the true carbonate alkalinity in a situation where non-carbonate sources of alkalinity are added requires the quantification of additional carbonate system parameters such as total dissolved inorganic carbon (DIC) along with pH and/or titration alkalinity (see, for instance, Example 5).

The present inventors have found a correlation between the dKH of the growth medium, and the mechanical and/or structural properties of the resulting coral. Specifically, it has been found that, by decreasing dKH, the porosity of the coral may be increased. Increased porosity can facilitate e.g. bone cell ingrowth, oxygen supply, and vascularization in newly formed bone tissue. However, as dKH is reduced, the rate of coral growth may decline. It has been found that this decline in growth rate may be compensated, at least in part, by increasing the pH of the growth medium above those normally encountered in seawater. In some cases, pH can be controlled independently of dKH, for instance, by selecting appropriate alkalis, such as hydroxides. For example, pH may be controlled at least in part by the addition of e.g. hydroxide. Hydroxide may be used to alter pH without altering the total amount of carbonate and bicarbonate in the growth medium.

Like many marine calcifiers, coral nucleates and grows CaCO₃ crystals within compartments that are isolated from the external seawater. Within these compartments, corals can modify, regulate and control conditions, including the carbonate chemistry of the calcifying fluid to enable CaCO₃ precipitation to occur. The conditions within the coral's calcifying region during active calcification, therefore, are very different from those of its external environment. Nonetheless, it has surprisingly been found that, by manipulating the pH of the surrounding growth medium to above natural seawater levels, the rate of calcification can be increased. Without wishing to be bound by theory, this is believed to be because, by increasing the external pH, the saturation state of aragonite (calcium carbonate) is increased, thereby reducing the thermodynamic burden on the coral in spite of low dKH. Furthermore, we believe that calcification is made more thermodynamically favourable due to the increased ease with which corals can remove the protons produced during calcification in their calcifying region out into the surrounding medium when the H⁺ concentration is relatively low in the surrounding medium due to high pH.. Accordingly, while calcification e.g. at low dKH may result in relatively low density structures, calcification can nevertheless occur at a desirable rate.

In some examples, dKH and pH may be controlled to provide the coral scaffold with a desirable balance between permeability and mechanical strength. As discussed above, permeability can facilitate e.g. bone ingrowth, oxygen supply, and vascularization in newly formed bone tissue. Excessive permeability, however, can compromise mechanical strength, which may be necessary for the formation of a matrix having sufficient load-bearing characteristics to reduce the risk of e.g. fracture at the grafting site before bone growth is complete. In some examples, the balance between mechanical strength and porosity may also influence the way in the bone graft substitute is resorbed by the body. While highly porous scaffolds may be easier to resorb, a risk of fracture may arise if resorption occurs too rapidly, for example, due to poor or incomplete remodelling of the bone.

In some examples, the pH of the growth medium may be maintained at a pH of 8.3 to 8.8. Preferably, the pH of the growth medium is maintained at a pH of 8.4 to 8.6.

The pH of the growth medium may be controlled by monitoring the pH of the growth medium and maintaining the pH within target values. The pH of the growth medium may be controlled and maintained by the addition of the hydroxides of group I or group II metals. For example, the pH of the growth medium may be maintained by the addition of sodium hydroxide and calcium hydroxide.

In some examples, pH may be controlled using a pH electrode connected to a controller, with specific set point for pH. As the pH drops below a target value, an aqueous stock solution of e.g. sodium hydroxide may be administered via a peristaltic pump until the pH is brought back into the target range.

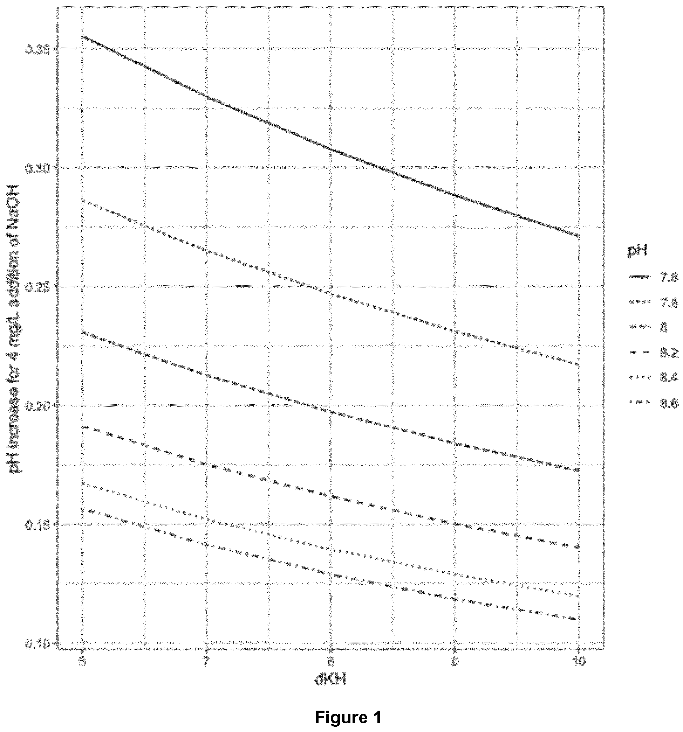

In some examples, the amount of e.g. metal hydroxide required to be dosed to raise the pH by a given value (e.g. to maintain pH at a set point against the biological processes which exert a downward pressure on pH) can be calculated by accounting for dKH. For example, the amount of e.g. metal hydroxide required to be dosed to raise the pH by a given value (e.g. to maintain pH at a set point against the biological processes which exert a downward pressure on pH) can be calculated by accounting for the buffering of seawater by the carbonate system. Figure 1 summarizes examples of such calculations (calculated using the *seacarb* package (https://cran.r-project.org/web/packages/seacarb/seacarb.pdf)) by demonstrating the rise in pH units resulting from an addition of 0.4 mg of NaOH per litre of coral growth medium, where the growth medium has the salinity and chemical composition of standard seawater and a temperature to 25 degrees Celcius.

It can be seen from Figure 1 that the magnitude of the change in pH for a given NaOH input may be sensitive to the starting pH and the dKH of the system but that the magnitude of the addition of 0.4 mg/L of NaOH has the approximate effect of increasing pH by around 0.1 pH units at pH >8.2. Scaling this up to e.g. a 500L tank, for instance, an increase in pH of around 0.1 units would typically therefore require an addition of 0.4 x 500 = 200 mg or 0.2 g of NaOH. This could be achieved, for example by the addition of 200ml of a 1 g per litre stock solution of NaOH.

In some examples, the carbonate hardness, dKH, may be monitored and maintained at target values. For instance, the carbonate hardness, dKH, of the growth medium may be maintained at less than 8. Preferably, dKH may be maintained at 7 or less. More preferably, dKH may be maintained at 6.5 or less. Without wishing to be bound by theory, the lower the dKH, the greater the porosity of the resulting coral.

In some examples, the method may comprise feeding:
(i) group I or group II metal hydroxide, and
(ii) bicarbonate
to the growth medium.

In some examples, the method may further comprise measuring or monitoring the pH and dKH of the growth medium and feeding (i) hydroxide, and (ii) bicarbonate to the growth medium to maintain pH and dKH within a target range.

In some examples, the method may further comprise feeding metal ions into the growth medium e.g. to facilitate incorporation of such ions into the coral structure, where these ions are known to play a beneficial role in bone formation. In some examples, the method may comprise feeding zinc, barium, magnesium and/or strontium ions into the growth medium. The amounts of e.g. zinc, barium, magnesium and/or strontium ions may be adjusted to facilitate the incorporation of such metals in target amounts relative to the total amount of calcium in the coral. In some examples, the method comprises controlling the amounts of zinc, magnesium, barium and/or strontium ions relative to calcium ions in the growth medium. In some examples, the method comprises controlling pH to vary the relative deposition rates of zinc, magnesium, barium and/or strontium relative to calcium in the coral.

In some examples, the concentration of metal ions, such as zinc (Zn), barium (Ba), strontium (Sr), barium (Ba) and/or magnesium (Mg) ions in the growth medium may be controlled to manipulate the incorporation of such ions into the coral structure. The metal ions may be introduced into the growth medium as metal compounds, such as metal salts and/or hydroxides. In some instances, the metal compound may be selected to allow metal ions to be introduced without altering the pH of the growth medium. For example, metal halides (e.g. chlorides, bromides, iodides) may be used. Alternatively, the metal compound may be selected to allow metal ions to be introduced while at the same time altering pH. For example, metal hydroxides may be used. In some instances, the compound may be selected to allow metal ions to be introduced without altering the total concentration of carbonate and bicarbonate in the growth medium.

In some examples, the method may further comprise Zn, Mg, Ba and Sr being added to the growth medium in the form of hydroxides (additionally raising the pH as per above) or as other soluble salts of Zn, Mg, Ba and Sr which have little or no effect on pH e.g. chlorides.

In some examples, a combination of two or more of pH, dKH, and metal ion concentration can be used to produce a coral based bone graft substitute of the desired physical and chemical properties.

Mechanical, structural and in some cases chemical and bioactive properties can be modified through (e.g. independent) control of the pH and dKH of the growth medium. In some cases soluble metal salts (e.g. Zn, Mg, Ba, Sr, Ca) could be added to the medium in varying ratios to further control the properties of the coral. Examples of properties that can be controlled include porosity, density, compressive strength, tensile strength, chemical composition, biomineralization, osteogenic differentiation and osteoblastic cell attachment.

Preferably, the coral scaffold comprises calcium carbonate. The coral scaffold may comprise at least 85 weight %, preferably at least 90 weight %, more preferably at least 95 weight % calcium carbonate. In some examples, the coral scaffold comprises at least 98 weight %, preferably at least 99 weight % calcium carbonate. The coral scaffold may consist essentially of calcium carbonate.

In some examples, at least 95 weight % of the calcium carbonate is present in the aragonite crystalline phase. In some examples, at least 99 weight % of the calcium carbonate is present in the aragonite crystalline phase. For instance, at least 99.5 weight % or at least 99.7 weight % of the calcium carbonate may be present in the aragonite crystalline phase. Crystallinity of the calcium phase may be important, as higher levels of crystallinity may improve the degradability of the bone graft substitute *in vivo.*

In some examples, the coral scaffold may comprise at least 85 weight %, preferably at least 90 weight %, more preferably at least 95 weight % calcium carbonate and at least 95 weight % of the calcium carbonate is present in the aragonite crystalline phase. Preferably, the coral scaffold comprises at least 98 weight %, preferably at least 99 weight % calcium carbonate and at least 99 weight % of the calcium carbonate is present in the aragonite crystalline phase. For instance, at least 99.5 weight % or at least 99.7 weight % of the calcium carbonate may be present in the aragonite crystalline phase.

The coral scaffold may be resorbable *in vivo.*

The method of the present invention may, in some instances, further comprise treating the coral scaffold to convert at least part of the coral scaffold to hydroxyapatite. The bone graft substitute, therefore, may be formed of a coral scaffold that comprises or consists essentially of hydroxyapatite.

In the method of the present invention, the devitalised coral may be sized in any suitable manner to form the coral scaffold. For example, the devitalised coral may be ground into a powder or broken into pieces or granules. Alternatively, the devitalised coral may be cut into wedges or blocks to form the bone graft substitute. The bone graft substituted may be an osteoconductive matrix, an osteoconductive scaffold and a bone void filler.

### Growth Medium

As described above, the method of the present invention comprises growing coral in a growth medium. The coral may be a calcifying species of coral. Any suitable genera or species of coral may be grown. Examples of suitable genera include *Pocillopora, Montipora, Favites, Favia, Porites, Goniastrea, Acanthestrea, Acropora, Alvepora, Fungia, Galaxea, Hydnophora, Millepora, Seriatopora, Stylophora, Sinularia, Alcyoniidae* and/or any coral of the order *Scleractinia.* For example, suitable species include *Pocillopora damicornis* and *Montipora digitata.* Preferably, species that can be grown in captivity in a mesocosm are selected.

The coral may be grown in any suitable growth medium. The growth medium may be an aqueous medium. The aqueous medium may be a saline solution. Examples of suitable growth media include freshwater, seawater and mixtures thereof. In some examples, a mixture of seawater and freshwater may be employed. In other examples, the growth medium may comprise a saline solution formed by dissolving sodium chloride and, optionally, other salts in water. The water may be filtered prior to use. In some examples, the growth medium may be circulated or agitated. This circulation or agitation may simulate the circulation of water that coral may be exposed to in its natural environment.

In some examples, the salinity of the growth medium may be controlled. Salinity levels may be varied depending on, for example, the species of coral grown. In some examples, the salinity may be controlled by varying the relative amounts of saltwater (e.g. seawater) and freshwater in the growth medium. Salinity may be controlled such that the specific gravity of the growth medium is from 1.0 to 1.1, preferably from 1.022 to 1.032 at 25 degrees C. In some examples, the salinity may be controlled from 1.024 to 1.026 at 25 degrees C, for instance, at about 1.025. In some examples, the method comprises controlling salinity within ± 10%, for example, ± 8%, ± 6%, ± 5%, ± 4%, ± 3%, ± 2%, ± 1%, ± 0.5% of a target specific gravity value. The target specific gravity may be 1, for example, 1.02 or 1.03.

In the method of the present invention, the growth medium has a carbonate hardness, dKH, of 10 or less. Carbonate hardness is a measure of the water hardness caused by the presence of carbonate (CO₃²⁻) and bicarbonate (HCO₃⁻) ions. The carbonate hardness is indicative of the extent to which the growth medium is buffered with respect to changes in pH.

In some examples, the carbonate hardness, dKH, of the growth medium may be maintained at less than 8. Preferably, dKH may be maintained at 7 or less. More preferably, dKH may be maintained at 6.5 or less. Without wishing to be bound by theory, the lower the dKH, the greater the porosity of the resulting coral.

For example, the dKH of the growth medium may be maintained at 10 or less for at least 6 hours, preferably, at least 12 hours or at least 18 hours. In some instances, the dKH of the growth medium may be maintained at 10 or more for at least a day, preferably at least 3 days, more preferably at least a week and yet more preferably at least a month. In preferred instances, the dKH of the growth medium may be maintained at 10 or less for at least 2 months, for example, at least 3 months. In some instances, the dKH of the growth medium may be maintained at 10 or less until the coral is collected.

In some examples, the dKH of the growth medium is controlled at a dKH value of 10 or less within ± 2 dKH units. In some examples, the dKH of the growth medium is controlled at a dKH value of 10 or less within ± 1.5 dKH units, preferably within ± 1 dKH units or more preferably within ± 0.5 dKH units .

The dKH of the growth medium may be varied by varying the amount of carbonate and/or bicarbonate in the growth medium. For example, carbonate and/or bicarbonate may be added to the growth medium to increase the dKH of the medium. In some examples, the growth medium may be dosed with a solution of carbonate and/or bicarbonate. For example, the carbonate and/or bicarbonate may be provided as an alkali metal salt, for instance, a sodium salt. In some examples, the growth medium may be dosed with a solution of sodium carbonate at intervals to maintain the dKH of the growth medium at 10 or less. Dosing may be performed as a bulk powder or as a stock solution.

In some examples, dKH may be monitored. dKH may be measured and/or monitored by any suitable method. For example, the dKH may be measured by manual visual titration, a manual colorimetric method or using an automatic pH electrode. In some examples, the dKH may be monitored continuously during the period of coral growth.

In addition to controlling the dKH, the concentration of certain elements in the growth medium may also be controlled. For example, the growth medium may be dosed with solutions of one or more salts, for example, metal salts. Examples of suitable salts include salts of magnesium, sodium, calcium, zinc and strontium. One or more of these salts may be employed. In some instances, metal cations present in the salts may be incorporated into the structure of the growing coral and/or otherwise promote coral growth. In some examples, the method comprises controlling the concentration of one or more metal ions in the growth medium by monitoring the concentration of such metal ions and dosing metal salts into the growth medium to maintain metal ion concentrations within target levels. The metal salts (e.g. as metal salt solutions) dosed into the growth medium may be selected, such that the concentration of metal ions may be controlled without substantially altering pH, dKH or the overall concentration of bicarbonate and carbonate in the growth medium. For example, the coral may incorporate metal ions, for instance, metal ions selected from magnesium, calcium, strontium, zinc and mixtures thereof. Suitable salts include chlorides, for example, magnesium chloride, calcium chloride and strontium chloride. Other suitable salts include phosphates, for example, sodium phosphate and hydroxides, for example calcium hydroxide. In some instances, anions present in the salts may be incorporated into the structure of the growing coral, or otherwise promote coral growth. As an example, phosphate ions present in phosphate salts (e.g. sodium phosphate) may facilitate growth of the coral skeleton.

In view of how the coral skeleton is enriched relative to calcium in coral grown under normal conditions of dKH, pH and the relative solubilities of group II and transition metal carbonates at different pH values (see examples), it may be possible to simultaneously control the porosity, growth rate and the relative and total abundances of group II and transition metal carbonates (e.g. zinc, barium, magnesium and/or strontium carbonates) incorporated into the coral skeleton by controlling the pH (e.g. through addition of hydroxide ions), dKH (through addition of bicarbonate ions) and the metal:calcium ion ratio during dosing of any metal ions which are desired to be included into the coral skeleton.

The growth medium may also be dosed with, for example, iodine. The coral may use iodine for the synthesis of pigments, which may allow them to adapt to varying light conditions and provide their tissue with protection from UV radiation. The amount of iodine in the growth medium may be controlled within prescribed limits. In some examples, it may be possible to dose iron salts in the growth medium. It has been found, however, that, in some circumstances, iron can increase the rate of nitrification.

The amount of salt(s) and other additives added to the growth medium may be controlled within narrow limits. Accordingly, solutions of the salts/additives may be dosed at predetermined amounts into the growth medium.

In some examples, the concentration of calcium salts (e.g. calcium chloride) may be controlled in the growth medium in an amount of 200 to 500 mg/l, preferably, 350 to 450 mg/l.

In some examples, the concentration of strontium salt (e.g. strontium chloride, strontium hydroxide) may be controlled in the growth medium in an amount of 0 to 12 mg/l, preferably, 6 to 9 mg/l.

In some examples, the concentration of iodine may be controlled in the growth medium in an amount of 25 to 250 mg/l, preferably, 100 to 200 g/l.

In some examples, the amount of phosphate (e.g. sodium phosphate) may be controlled from 0 to 3 mg/l, for example, 0.001 to 1 mg/l.

In some examples, the amount of iron may be controlled from 0 to 3000 mg/l. Preferably, iron concentrations are limited to below 5 mg/l, preferably, below 1 mg/l. More preferably, no iron is added to the growth medium.

The growth medium may also be dosed with ammonium salts. For example, ammonium chloride may be used. Coral has a symbiotic relationship with a dinoflagellate algae of the genus *Symbiodinium.* This symbiosis is based on mutual nutrient exploitation, with corals providing shelter and inorganic nutrients to their algal partners, while *Symbiodinium* supply their coral hosts with photosynthates that can meet at least part of the corals' energy requirements. By maintaining ammonium levels in the growth medium, it is possible to support algal growth, which, in turn, facilitates the growth of the coral. In some examples, ammonium levels are maintained at a concentration of 0 to 1.5 ppm, preferably about 0.5 ppm.

Ammonium levels may be maintained by dosing ammonium salts into the growth medium e.g. periodically or continuously. Ammonium levels may be maintained at about 0.5 ppm for e.g. a day. Dosing may be carried out as a bulk powder or as a stock solution.

Where dosing solutions are added to the growth medium, dosing may be carried out manually e.g. at periodic intervals during the growth of the coral. Alternatively, dosing may be carried out automatically e.g. using a peristaltic pump. Dosing may be carried out using a stock solution. The dosing strategy may be based on altering the amount of a given element in such as way as to maintain a desired elemental ratio to calcium, which is regularly dosed to enhance growth. For example, a strontium to calcium ratio similar to or greater than that found in natural seawater might be used.

In some instances, live rock may be placed in the growth medium. Coral may anchor to the rock. The rock may also provide a site for nitrifying bacteria within the mesocosm.

The growth medium may be cycled prior to use to ensure that it is stable with nitrifying bacteria. Cycling may be performed according to methods that are well-known in the art.

During the period of coral growth, the temperature of the growth medium may be controlled. Suitable temperatures range from 10 to 32 degrees C, preferably 19 to 32 degrees C. Preferably, the temperatures are controlled within20 to 27 degrees C, more preferably at about 25 degrees C.

The growth medium may also be subjected to a controlled amount of light on a predetermined cycle as required. The growth medium may be irradiated with light for 4 to 20 hours a day, for example, 6 to 15 hours a day, preferably 8 to 12 hours a day.

It may take at least one month, for example, two to six months before coral is ready for harvesting/collection. In some instances, it may take three to four months before the coral is ready for harvesting. In some examples, the coral may be considered to be ready for harvesting once it reaches at least 150%, preferably, at least 180% or at least 200% of its original volume. Once ready for harvest, the coral may be collected using known methods.

### Devitalising Coral

The collected coral may be devitalised using any suitable method. For example, the coral may be devitalised by treatment with an oxidizing agent, for instance, hypochlorite (e.g. sodium hypochlorite). In one example, the coral may be treated with a solution of hypochlorite for a predetermined length of time. Suitable time periods range from 3 to 50 hours, for example, 5 to 40 hours. If required, the devitalised coral may then be rinsed with water (e.g. deionized water). The collected coral may be dried. Drying may be performed at elevated temperatures. Suitable temperatures range from 50 to 190 degrees C, for instance, 80 to 100 degrees C. In some instances, the collected coral may be subjected to depyrogenation. The devitalised coral may then be sized using any suitable technique. For example, the devitalised coral may be ground into a powder, or broken into pieces. Where the devitalised coral is broken into pieces, the pieces may measure 0.5 to 5 mm, preferably, 1 to 2 mm across. Alternatively, the devitalised coral may be cut into blocks or wedges, depending on the nature of the bone graft substitute produced.

Where the devitalised coral is sized to form particles of a coral scaffold, sizing may be carried out by crushing or milling. Any suitable crushing or milling technique may be used. For instance, the devitalised coral may be milled.

### Other Treatments

Once sized, the devitalised coral may be used to form the coral scaffold of the bone graft substitute. In some examples, the sized coral may be used as the coral scaffold after, for example, cleaning and disinfection.

In other examples, the coral may be treated prior to use as the coral scaffold. For example, the coral may be treated to convert at least part of the calcium carbonate in the coral to hydroxyapatite. Any suitable method for converting the calcium carbonate in the coral to hydroxyapatite may be employed. For example, the calcium carbonate may be treated by hydrothermal treatment to produce hydroxyapatite. In some examples, the coral may be treated with phosphoric acid (H₃PO₄) a dihydrogen phosphate salt (e.g. ammonium dihydrogen phosphate, NH₄H₂PO₄) to produce hydroxyapatite. The reaction may occur in the presence of water and at elevated temperatures. Suitable temperatures range from, for example, 160 to 220 degrees C. Suitable pressures range from 6 to 10 MPa, for example, 8 MPa. While the conversion of calcium carbonate to hydroxyapatite may be useful for certain applications, for other applications, it may be preferable for the calcium carbonate to remain unconverted. In some examples, coral scaffolds comprising calcium carbonate may be preferable for use in the manufacture of resorbable bone grafts. In some examples, the coral scaffold is used without treating the scaffold to convert the calcium carbonate to hydroxyapatite.

### Porosity

The coral scaffold may have a porosity of at least 15%, for example, at least 18% or at least 20%. In some examples, the porosity may be at least 21%, at least 25%. at least 30%, at least 40%, at least 50%, or at least 60%. In some examples, the coral scaffold may have a porosity of at least 70%, at least 75%, and at least 80% In some examples, the coral scaffold may have a porosity of at least 85%, at least 90%. The coral scaffold may have a porosity of up to 98%, for example, up to 95%. In some instances, the coral scaffold may have a porosity of 15 to 98%, 18 to 80%, 20 to 70% or 21 to 60%. In some examples, the coral scaffold may have a porosity of 70 to 95%, for example, 75 to 90% or 80 to 85%. These porosities may reflect the volume porosity of the coral scaffold. For instance, where the coral scaffold takes the form of an individual unit (e.g. wedge or block), the porosity may be indicative of the volume or pores within the unit (e.g. wedge of block). Similarly, where the coral scaffold takes the form of particles, the porosity may be indicative of the volume of the pores within each particle of the coral scaffold. This total pore volume may reflect the total pore volume of the devitalised coral. This total pore volume may reflect the "internal" pore volume of the coral scaffold. Alternatively, the porosities may also reflect the porosity of voids between individual particles of the coral scaffold. For example, where the coral scaffold comprises particles, the volume porosity may include the volume of pores within each particle and the volume of the voids formed between particles.

In some examples, the coral scaffold comprises particles. The porosity of the coral scaffold particles may be at least 15%, for example, at least 18% or at least 20%. In some examples, the porosity may be at least 21%, for example, at least 25%, at least 30%, at least 40%, at least 50%, or at least 60%. In some examples, the coral scaffold particles may have a porosity of least 70%, preferably at least 75%, and yet more preferably at least 80%. In some examples, the coral scaffold particles may have a porosity of at least 85%, preferably 90%. The coral scaffold particles may have a porosity of up to 98%, preferably up to 95%. In some instances, the coral scaffold particles may have a porosity of 15 to 98%, 18 to 80%, 20 to 70% or 21 to 60%. In some instances, the coral scaffold particles may have a porosity of 70 to 95%, for example, 75 to 90% or 80 to 85%. These porosities may reflect the porosity within each individual particle, or the porosity within each individual particle and the void volume before the particles.

As discussed above, the porosity of the coral scaffold is a relevant parameter with respect to its application as a bone graft substitute. In some instances, porosity can affect bone growth into the grafted area and, thus, graft integration and healing. By carrying porosity (e.g. increasing porosity) of the coral scaffold, the permeability of the bone graft substitute may be improved.

This may improve the amount of bone ingrowth into the scaffold. Varying (e.g. increasing) porosity may also provide greater space for nutrient and oxygen supply, and further vascularization in newly formed bone tissue. In some instances, varying porosity (e.g. decreasing porosity) may also result in a resorbable structure that can be tailored to suit requirements

Porosity may be measured by any suitable method.

In some examples, the porosity of the sample may be measured by mercury porosimetry. Mercury porosimetry may be used to measure the porosity within the coral scaffold. Mercury porosimetry is used to measure the porosity of a material by applying controlled pressure to a sample immersed in mercury.

External pressure is required for mercury to penetrate into the pores of a material due to high contact angle of mercury. The amount of pressure required to intrude into the pores is inversely proportional to the size of the pores. The larger the pore the smaller the pressure needed to penetrate into the pore. A mercury porosimeter can generate volume and pore size distributions from the pressure versus intrusion data generated by the instrument using the Washburn equation. A mercury porosimeter can also be used to provide the total pore size area of a sample. Mercury porosimetry may be useful for determining the porosity of the coral scaffold, for instance, within individual particles of the coral scaffold.

The porosity of the coral scaffold may be determined using known methods described in e.g. ISO 23145-1:2007 or ASTM UOP 578-11. In one example, the porosity may be measured by inserting a volume of the coral scaffold into a test container having a known volume. The test container may be tapped several times (e.g. 1000 times) using, for example, a pneumatic device to ensure that the coral scaffold settles into the test container. Excess coral scaffold can be removed from the top of the container to ensure that the container is filled. By dividing the mass of the filled container by the known volume of the container, the tapped density, ρ tapped, may beobtained. The porosity (volume porosity), P, may be calculated from the tapped density as follows:
$P = \left[1-\left(ρ tapped/ρ theoretical\right)\right] \times 100$ where ρ theoretical is the theoretical density of the coral scaffold material (e.g. aragonite, ρ theoretical = 2.93 g/cm³).

This porosity determined from the tapped and theoretical densities may be indicative of the porosity within and between the particles of the coral scaffold.

The coral scaffold may comprise pores that are 1 to 800 microns in size. The coral scaffold may have a heterogeneous pore size. In some examples, the coral scaffold may comprise pores that are 100 to 500 microns in size, preferably to 325 microns in size. The coral scaffold may have pores with suitable sizes to facilitate cell adhesion, aggregation and proliferation, while at the same time providing sufficient space for vascularization for adequate nutrient and oxygen supply. The pores may be formed within the structure of the coral rather than between individual particles of the coral scaffold.

The coral scaffold may have a bulk density of at least 1.9 g/cm³, for example, 2.00 to 2.20 g/cm³, preferably 2.02 to 2.18 g/cm³.

### Calcium and Calcium Carbonate content

The coral scaffold may have a calcium content of greater than at least 35 weight %, preferably at least 37 weight %. In some examples, the coral scaffold may contain 35 to 45 weight % calcium, preferably 37 to 40 weight % calcium.

Preferably, the coral scaffold comprises calcium carbonate. The coral scaffold may comprise at least 85 weight %, preferably at least 90 weight %, more preferably at least 95 weight % calcium carbonate. In some examples, the coral scaffold comprises at least 98 weight %, preferably at least 99 weight % calcium carbonate. The coral scaffold may consist essentially of calcium carbonate.

In some examples, at least 95 weight % of the calcium carbonate is present in the aragonite crystalline phase. In some examples, at least 99 weight % of the calcium carbonate is present in the aragonite crystalline phase. For instance, at least 99.5 weight % or at least 99.7 weight % of the calcium carbonate may be present in the aragonite crystalline phase. In some examples, a small amount of calcite may be present. For example, calcite may form up to 1 weight %, preferably up to 0.5 weight %, more preferably up to 0.3 weight % of the calcium carbonate present in the coral scaffold. In some instances, the calcium carbonate consists essentially of calcium carbonate present in the aragonite and/or calcite phase. Crystallinity of the calcium phase may be important, as higher levels of crystallinity may improve the degradability of the bone graft substitute *in vivo.* Phase quantification may be carried out using any suitable method, for example, by X-ray diffraction as outlined in, for instance, ISO 13779-3:2018.

In some examples, the coral scaffold may comprise at least 85 weight %, preferably at least 90 weight %, more preferably at least 95 weight % calcium carbonate and at least 95 weight % of the calcium carbonate is present in the aragonite crystalline phase. Preferably, the coral scaffold comprises at least 98 weight %, preferably at least 99 weight % calcium carbonate and at least 99 weight % of the calcium carbonate is present in the aragonite crystalline phase. For instance, at least 99.5 weight % or at least 99.7 weight % of the calcium carbonate may be present in the aragonite crystalline phase.

In addition to calcium, other metals may be present in the coral scaffold. For example, the coral scaffold may comprise sodium, magnesium and/or strontium. Where magnesium is present, magnesium may be present in an amount of up to 2000 mg/kg, for instance, 1000 to 1500 mg/kg, preferably 1100 to 1300 mg/kg. Where sodium is present, sodium may be present in an amount of up to 8000 mg/kg, for example, 4000 to 7000 mg/kg, preferably 5000 to 6000 mg/kg. Where strontium is present, strontium may be present in an amount of up to 9000 mg/kg, for example, 65000 to 8500 mg/kg or 7000 to 8000 mg/kg.

As mentioned above, the coral scaffold is resorbable *in vivo.* When the coral scaffold is dissolved in e.g. a TRIS (trisaminomethane) buffer solution, the pH of the resulting solution is substantially unchanged even after 24, 48 or 72 hours. The maximum deviation from the initial pH value may be less than 0.3, preferably less than 0.2.

### Bone Graft Substitute

The coral scaffold may be sized using any suitable method to provide the bone graft substitute. For example, the coral scaffold may be ground into a powder or particles for use as the bone graft substitute. Alternatively, the coral scaffold may be cut into blocks or wedges as required for use as a bone graft substitute. The size and shape employed may depend on the would requiring treatment.

In some examples, the coral scaffold may be sized as particles or "granules". The particles may have a particle size of 0.1 to 5 mm, for example, 0.5 to 3 mm. Preferably, the particle size may be 1 to 2 mm. In some examples, at least 70 weight %, more preferably at least 80 weight % or at least 90 weight % of the particles may have a particle size of from 1 to 2 mm.

The coral scaffold may form a bone graft substitute for use as an osteoconductive matrix. In some examples, the coral scaffold may be used as an osteoconductive matrix for cancellous bone tissue.

The coral scaffold may be used as bone graft substitute for use as a bone void filler. Such a substitute may be used to fill gaps and voids in bone, for example, those caused by trauma or disease. Such bone graft substitutes may be injectable and/or mouldable to conform to the wound site.

The bone graft substitute may be suitable for promoting soft tissue in growth, for instance, collagen in-growth.

The bone graft substitute may have a porosity of at least 15%, for example, at least 18% or at least 20%. In some examples, the porosity may be at least 21%, at least 25%, at least 30%, at least 40%, at least 50%, or at least 60%. In some examples, the bone graft substitute may have a porosity of at least 70%, preferably at least 75%, and yet more preferably at least 80%. In some examples, the bone graft substitute may have a porosity of at least 85%, preferably 90%. The bone graft substitute may have a porosity of up to 98%, preferably up to 95%. In some instances, the bone graft substitute may have a porosity of 15 to 98%, 18 to 80%, 20 to 70% or 21 to 60%. In some instances, the bone graft substitute may have a porosity of 70 to 95%, for example, 75 to 90% or 80 to 85%.

These porosities may reflect the volume porosity of the bone graft substitute. For instance, where the bone graft substitute takes the form of an individual unit (e.g. wedge or block), the porosity may be indicative of the volume or pores within the unit (e.g. wedge or block). Similarly, where the bone graft substitute takes the form of particles, the porosity may be indicative of the volume of the pores within each particle of the bone graft substitute. This total pore volume may reflect the total pore volume of the devitalised coral. This total pore volume may reflect the "internal" pore volume of the bone graft substitute. Alternatively, the porosities may also reflect the porosity of voids between individual particles between particles of the bone graft substitute. For example, where the bone graft substitute comprises particles, the volume porosity may include the volume of pores within each particle and the volume of the voids formed between particles.

Preferably, the total pore volume reflects the "internal" pore volume of the bone graft substitute. Accordingly, where the bone graft substitute takes the form of particles, the porosity is indicative of the volume of the pores within each particle of the bone graft substitute.

In some examples, the bone graft substitute comprises particles. The porosity of the bone graft substitute particles may be at least 15%, for example, at least 18% or at least 20%. In some examples, the porosity may be at least 21%, at least 25%, at least 30%, at least 40%, at least 50%, or at least 60%. The porosity of the bone graft substitute particles may be at least 70%, preferably at least 75%, and yet more preferably at least 80%. In some examples, the bone graft substitute particles may have a porosity of at least 85%, preferably 90%. The bone graft substitute particles may have a porosity of up to 98%, preferably up to 95%. In some instances, the bone graft substitute particles may have a porosity of 15 to 98%, 18 to 80%, 20 to 70% or 21 to 60%. In some instances, the bone graft substitute particles may have a porosity of 70 to 95%, for example, 75 to 90% or 80 to 85%. These porosities may reflect the porosity within each individual particle, or the porosity within each individual particle and the void volume before the particles.

As discussed above, the porosity of the bone graft substitute is a relevant parameter for bioengineering applications. In some instances, porosity can affect bone growth into the grafted area and, thus, graft integration and healing. The porosity in the bone graft substitute can affect the permeability, affecting the amount of bone ingrowth. The porosity may also provide adequate space for nutrient and oxygen supply, and further vascularization in newly formed bone tissue. The porosity may also influence the mechanical strength of the bone graft substitute.

Porosity may be measured by any suitable method, including methods that are known in the art and those methods discussed above in relation to the coral scaffold.

The bone graft substitute may comprise pores that are 1 to 800 microns in size. The coral scaffold may have a heterogeneous pore size. In some examples, the bone graft substitute may comprise pores that are 100 to 500 microns in size, preferably 100 to 325 microns in size. The bone graft substitute may have pores with suitable sizes to facilitate cell adhesion, aggregation and proliferation, while at the same time providing sufficient space for vascularization for adequate nutrient and oxygen supply. The pores may be formed within the structure of the coral rather than between individual particles of the bone graft substitute.

The bone graft substitute may have a bulk density of at least 1.9 g/cm³, for example, 2.00 to 2.20 g/cm³, preferably 2.02 to 2.18 g/cm³.

In some examples, the bone graft substitute may be treated with a pharmaceutical composition, protein, growth factor or cells (e.g. stem cells) to facilitate bone growth.

In some examples, the bone graft substitute may have a compressive strength of 1.5 to 6 MPa, for instance, 3 to 5 MPa.

### Example1

Coral of the species *Pocillopora damicornis , Monitpora capricornis and Stylophora Milka,* were grown in a growth medium comprising a mixture of seawater and freshwater. Seawater was sourced from the Atlantic ocean and filtered and brought up to temperature before use.

Freshwater was treated by reverse osmosis and maintained at an appropriate temperature prior to use. The mesocosm was cycled before any livestock was added to the system.

The mesocosm was controlled as shown in Table 1 below:

| Parameter | Target set point |
|---|---|
| Lighting/hours | 10 |
| Temperature/°C | 25 |
| Salinity/Specific Gravity | 1.025 |
| Carbonate hardness/dKH | 6.6 |
| Calcium/mg/I (based on weight of total salt) | 400 |

| | |
|---|---|
| Strontium/mg/I (based on weight of total salt) | 8 |
| lodine/mg/I | 60 |
| Iron/mg/l | 0 |
| Nitrate/mg/I | 1 |
| Phosphate/mg/l | 0.03 |

After approximately 3 months of growth, corals were collected from the mesocosm as whole units. The corals were then cut in half. One half was returned to the mesocosm for further growth, while the remaining half was devitalised by exposing the coral to a 5% solution of hypochlorite for 30 hours. The devitalised coral was then rinsed in deionized water for 30 hours and dried at 90 degrees C for 2 hours.

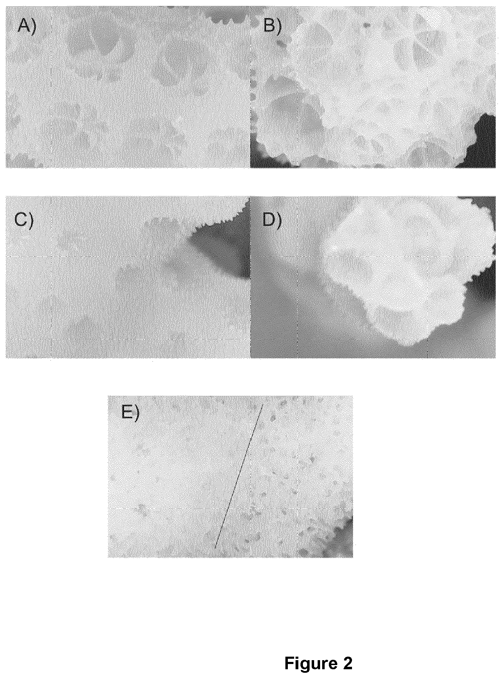

Microscopy images of the devitalised coral were compared to parts of the same corals grown at a dKH of >8. The coral grown at a dKH of 6.6 was observed to be more porous (Figure 2). However, the growth rate of the coral was found to be substantially slower than under normal growth conditions.

In Figure 2, A) and B) show *Stylophophora milka* grown at dKH 8.5 and <6.5, respectively; C) and D) show *Pocillopora damicornis* grown at dKH 8.5 and <6.5 respectively; E) show *Montipora capricornis* from Example 1 showing growth under normal conditions of dKH 8.5 (to the left of the image) and the growth under dKH <6.5 conditions (right of image); the line running across the image demarking approximately the transition point between growth under the 2 different dKH conditions.

### Example 2

By comparison of the X:Ca ratio (where X = strontium, zinc, magnesium, barium) of the coral to the X:Ca ratio of standard seawater, it may be possible to identify the enrichment of ions in the coral skeleton relative to calcium (e.g. Sreekumaran et al 1972, Giri et al., 2018). The so-called 'distribution factor', D for an ion, X, is calculated as the ratio of the abundance of X to the abundance of calcium in coral skeleton divided by the ratio of the abundance of X to the abundance of calcium in seawater (Giri et al., 2018).${D}_{X} = {\left(X/Ca\right)}_{CORAL} / {\left(X/Ca\right)}_{SEAWATER}$

Coral of the species *Pocillopra damicornis* was grown under normal conditions (Table below) and analysed by inductively coupled plasma atomic emission spectroscopy for elemental composition. It was found to have the following distribution factors, compared to the composition of standard seawater:

| Element | Distribution factor |
|---|---|
| Strontium | 0.61 |
| Magnesium | 0.0009 |
| Zinc | 0.24 |
| Phosphorous | 0.13 |
| Barium | 0.9 |

These distribution factors being less than 1 at first interpretation may suggest that coral is actively excluding these elements from its skeleton. It is certainly true that coral has a stronger preference for Calcium than any of the above elements. However, when compared to their ratios in seawater all of the above elements are found to be heavily enriched relative to sodium, one of the most abundant ions in seawater so it can be considered that all of the above elements as well as calcium are enriched relative to their abundance in the growth medium.

These results suggest that the incorporation of magnesium and strontium relative to calcium in coral skeletons is sensitive to the ratio of these metal ions to calcium in seawater and therefore by inference that increasing these ratios in a coral growth medium should allow the enhancement of these ratios in the coral skeleton.

To demonstrate this, coral of the genus *Pocillopra damicornis* were grown under the same conditions as above with the modification of Sr:Ca ratio in the dosing medium maintained such that the Sr concentration in the growth medium was periodically increased from 8ppm to 10ppm. The coral grown under these conditions was found by EDX (energy dispersive X-ray) analysis to have a molar ratio of Sr:Ca of 0.0126 vs under normal conditions (measured by ICP-AES as per the above) of 0.00913, the Sr:Ca ratio therefore having increased by ~37% for an approximately 25% increase in the dosing ratio.

The above evidence is strong evidence that control of the dosing ratio of elements relative to calcium into the growth medium is a feasible approach to controlling the elemental ratios of the coral based bone graft substitute.

### Example 3

This example demonstrates that pH can exert control over the incorporation of Zn, Mg, Ba and Sr into the coral skeleton and that the proportional uptake of the above mentioned elements relative to calcium is indeed pH dependent.

As the salts of strong bases and weak acids, the solubility of metal carbonates is pH dependent. The relationship between solubility and pH can be calculated as follows. $S = square root \left({K}_{SP}*\left(1+\left(\left[H+\right]/{K}_{2}\right)\right)\right)$ where S is the solubility in moles per litre, [H+] is the concentration of hydrogen ions (i.e. 10^{-pH}), K_{SP} is the solubility product of the metal carbonate and K₂ is the second dissociation constant of carbonic acid (i.e. the dissociation constant for bicarbonate).

K_{SP} for the metal carbonates of interest is given below

| | |
|---|---|
| Mg | 6.80e-06 |
| Ca | 3.40e-09 |
| Sr | 5.60e-10 |
| Ba | 2.60e-09 |
| Zn | 1.46e-10 |

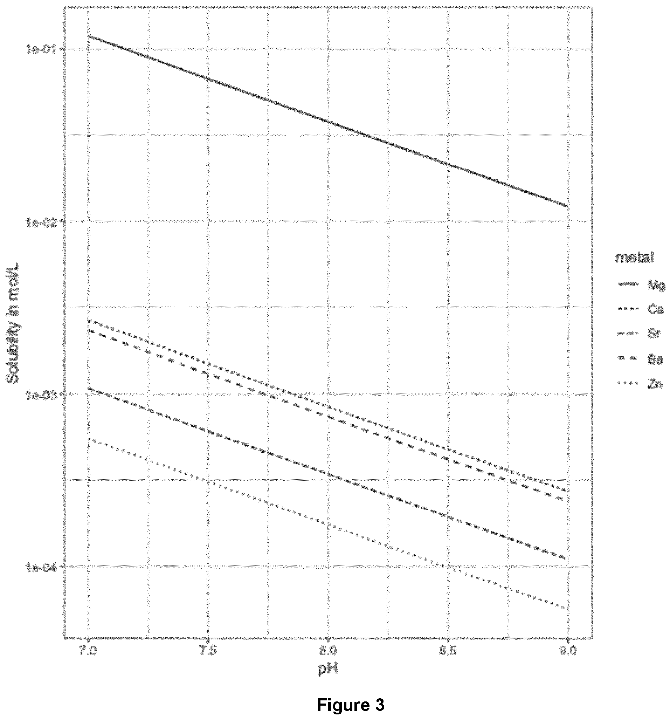

Using the data and equation above we calculate the pH - solubility relationship for the above metal carbonates that are of interest to bone formation (Figure 3).

Note that solubility in mol/kg and that the y axis is a logarithmic scale.

The calculations demonstrate that the ratios of the above metals are likely to change in coral skeleton due to the change in pH. For example, with increasing pH MgCO₃ becomes disproportionately less soluble compared to CaCO₃. At higher pH, Zn and Sr carbonates become relatively more soluble compared to calcium carbonate Therefore, assuming that coral calcification is sensitive to external pH and not wishing to be bound by theory we predict a pHdependent relationship of Mg:Ca, Sr:Ca, Zn:Ca and Ba:Ca ratios in coral grown under our controlled conditions.

### Example 4

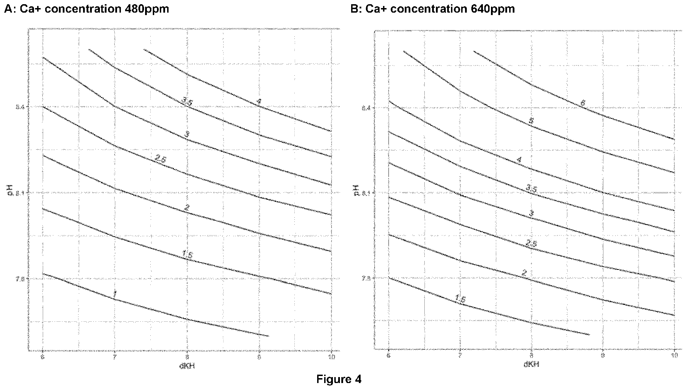

In this example we consider the sensitivity of the aragonite saturation state in a theoretical growth medium with properties identical to standard seawater. The saturation state is a measure of the thermodynamic tendency for aragonite to dissolve (saturation <1) or precipitate (saturation >1). Where the saturation state is higher it is easier for Coral to build its skeleton. Figure 4 presents the aragonite saturation state over a range of pH and dKH with panel a) showing these values for a typical calcium ion concentration in seawater of around 480 ppm (0.48g/L) and panel b showing the same for an elevated calcium ion concentration of 640 ppm.

This example demonstrates that at low dKH (which will tend to be associated with low pH unless intervention such as addition of NaOH is undertaken) the saturation state of aragonite tends to be low. This low dKH-low pH condition leads to a situation as in example 1 where coral grows slowly with enhanced porosity. The coral in this condition is challenged both by the lack of availability of carbonate ions for aragonite formation but also the low saturation state of aragonite making precipitation of aragonite skeleton thermodynamically difficult. By raising the pH at low dKH e.g. top-left region of panel a, it is possible relieve the thermodynamic pressure on the coral while still limiting its ability to make skeleton through the availability of carbonate ions. Therefore, coral can make skeleton of enhanced porosity at a faster rate of growth. Panel b further demonstrates the added thermodynamic benefit of elevated calcium ions in raising the saturation state of aragonite.

### Examole5

Corals of the species *Pocillopora damicornis, Turbinaria reniformis, Montipora capricornis and Stylophora milka* were grown in a growth medium comprising a mixture of seawater and freshwater. Seawater was sourced from the Atlantic ocean and filtered and brought up to temperature before use.

Freshwater was treated by reverse osmosis and maintained at an appropriate temperature prior to use. The mesocosm was cycled before any livestock was added to the system.

The mesocosm was controlled as shown in Table 1 below:

| Parameter | Target set point |
|---|---|
| Lighting/hours | 10 |
| Temperature/°C | 25 |
| Salinity/Specific Gravity | 1.025 |
| Calcium/mg/I (based on weight of total salt) | 400 |

| | |
|---|---|
| Strontium/mg/I (based on weight of total salt) | 8 |
| lodine/mg/I | 60 |
| Iron/mg/l | 0 |
| Nitrate/mg/I | 1 |
| Phosphate/mg/l | 0.03 |

1 Molar sodium hydroxide solution was dosed into the system via peristaltic pump to maintain the pH in the mesocosm within the range 8.2 to 8.4. dKH control was set to dose the mesocosm with sodium carbonate in the event that dKH fell below 6.5. In practice, the dKH measurement used relies on estimation of carbonate hardness by total alkalinity titration and therefore the apparent dKH was routinely above the set point and no dosing of carbonate solution was done throughout the experiment. Therefore the only carbonate available to the coral was that present in seawater added during water changes and any carbonate arising from the dissolution of CO₂ from the atmosphere above the mesocosm (which would be enhanced by sodium hydroxide addition but is still expected to be small compared to the amount of carbonate dosed under normal growth conditions and also less than that added in the first low dKH experiment described in example 1). Consequently the experiment was conducted at very low dKH (i.e. significantly less than 6.5); but the true value of dKH was unquantified.

After approximately 3 months of growth, the coral was collected as a whole unit. The coral was then cut in half. One half was returned to the mesocosm for further growth, while the remaining half was devitalised by exposing the coral to a 5% solution of hypochlorite for 30 hours. The devitalised coral was then rinsed in deionized water for 30 hours and dried at 90 degrees C for 2 hours.

Using visual estimation of linear extension over the growth period, the *Pocillopora damicornis* coral was observed to grow at a considerably higher rate than the experiment described in example 1, and all coral species were observed to have grown at a rate broadly similar to that in normal growth conditions in tanks where these species of corals are routinely grown and dKH is maintained at >8.

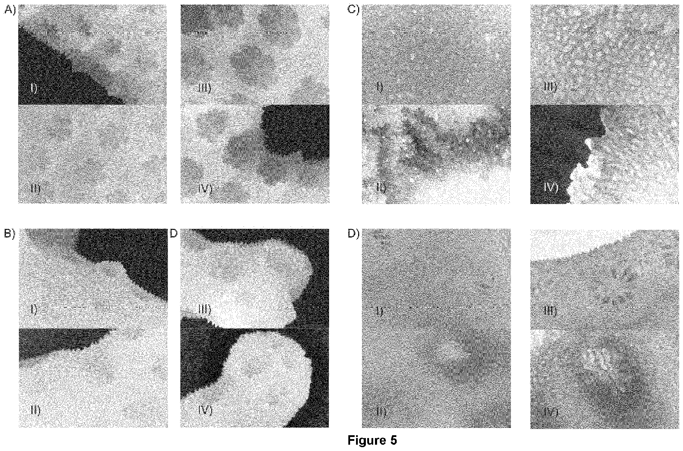

Visual and microscopic analysis of the coral grown in this experiment revealed a clear change in porosity and density of the skeletal material under the changed growth conditions. Specifically, large pores where polyps grew were larger and spaced closer together in branching corals (*S. milka* and *P*. *damicornis*) and interconnected pores were larger in plating coral species (*M. capricornis* and *T. reniformis*) and in all corals the newly grown material was more translucent to light under the low dKH-high pH conditions (see photo-microscopy in Fig. 5).

In Figure 5, all comparative images e.g. panels I and III, or II and IV for each coral species were taken at the same magnification. A) *Stylophora milka,* all images produced by reflective light microscopy. Panels I) and II) show parts of the coral grown under normal conditions (dKH >8) and III) and IV) show coral growth under test conditions (low dKH, high pH). B) *Pocillopora damicornis,* all images produced by reflective light microscopy. Panels I) and II) show parts of the coral grown under normal conditions (dKH >8) and III) and IV) show coral growth under test conditions (low dKH, high pH). C) *Montipora capricornis,* all images produced by backlit microscopy. I) underside and II) topside of coral under normal growth conditions; III) underside and IV) topside of coral under test conditions. D) *Turbinaria reniformis,* all images of topside of coral. I) reflective light and II) backlit images of coral grown under normal conditions; III) reflective light and II) backlit images of coral grown under test conditions. In all cases test conditions increase aspects of porosity evaluated by microscopy, relative to normal growth conditions.

In a future controlled growth experiment, an improved control system would be to use dKH monitoring to jointly dose sodium carbonate and sodium hydroxide at a selected ratio to ensure pH and dKH were controlled to within appropriate ranges or, alternatively, to monitor both titration alkalinity and pH simultaneously with total dissolved inorganic carbon and use a numerical model of the carbonate system to determine and control precisely the dKH and speciation of carbonate/bicarbonate in the growth medium.

### References:

Sharmila J. Giri, Peter K. Swart, Quinn B. Devlin, The effect of changing seawater Ca and Mg concentrations upon the distribution coefficients of Mg and Sr in the skeletons of the scleractinian coral Pocillopora damicornis, Geochimica et Cosmochimica Acta, Volume 222, 2018, Pages 535-549, https://doi.org/10.1016/j.gca.2017.11.011.
Inoue, M., Suwa, R., Suzuki, A., Sakai, K., and Kawahata, H. (2011), Effects of seawater pH on growth and skeletal U/Ca ratios of Acropora digitifera coral polyps, Geophys. Res. Lett., 38, L12809, doi:10.1029/2011GL047786.
Schleinkofer, N., Raddatz, J., Freiwald, A., Evans, D., Beuck, L., Rüggeberg, A., and Liebetrau, V.: Environmental and biological controls on NaCa ratios in scleractinian cold-water corals, Biogeosciences, 16, 3565-3582, https://doi.org/10.5194/bg-16-3565-2019, 2019.
SREEKUMARAN, C., & GOGATE, S. S. (1972). STUDIES ON MINERAL CONSTITUENTS OF SOME SPECIES OF CORALS. Current Science, 41(7), 241-244. http://www.jstor.org/stable/24075152.

## Claims

1. A method of manufacturing a coral scaffold for use as a bone graft substitute, the method comprising
growing coral in a growth medium,
removing at least a portion of the coral from the growth medium, and
devitalising coral removed from the growth medium,
wherein the pH of the growth medium is controlled at a pH of 8.3 or greater, and wherein the growth medium has a carbonate hardness, dKH, is controlled at less than 10.

2. A method as claimed in claim 1, wherein the dKH of the growth medium is maintained at less than 10 and pH of the growth medium is maintained at a pH of 8.3 to 8.8.

3. A method as claimed in claim 2, wherein the pH of the growth medium is maintained at a targeted pH of 8.4 to 8.6.

4. A method as claimed in any one of the preceding claims, wherein the pH of the growth medium is controlled by the addition of a group I or group II metal hydroxide.

5. A method as claimed in any one of the preceding claims, wherein the pH of the growth medium is controlled by the addition of sodium hydroxide.

6. A method as claimed in claim 1, wherein the growth medium has a carbonate hardness, dKH, that is controlled at less than 8.

7. A method as claimed in any one of the preceding claims, wherein the growth medium has a carbonate hardness, dKH, that is controlled at 7 or less.

8. A method as claimed in any one of the preceding claims, wherein the growth medium has a carbonate hardness, dKH, that is controlled at 6.5 or less.

9. A method as claimed in any one of the preceding claims, wherein the growth medium has a carbonate hardness, dKH, that is controlled at less than 6.

10. A method as claimed in any one of the preceding claims, which comprises feeding:
(i) ammonium hydroxide and/or alkali metal hydroxide, and
(ii) bicarbonate
to the growth medium.

11. A method as claimed in claim 10, which further comprises measuring pH and dKH of the growth medium and feeding (i) group I, group II or transition metal hydroxide(s), and (ii) bicarbonate to the growth medium to maintain pH and dKH within a target range.

12. A method as claimed in any one of the preceding claims, which further comprises feeding zinc, magnesium and/or strontium chloride into the growth medium.

13. A method as claimed in any one of the preceding claims where phosphate is added to the growth medium in proportion with calcium, thereby controlling the phosphate content of the resulting bone graft substitute.

14. A method as claimed in any one of the preceding claims, wherein dKH is controlled independently of pH.

15. A method as claimed in any one of the preceding claims, wherein the incorporation of strontium, zinc and/or magnesium in the coral is controlled by monitoring the concentration of strontium, zinc and/or magnesium in the growth medium and adjusting the concentration of strontium, zinc and/or magnesium within target limits, and optionally wherein the pH of the growth medium is adjusted to enhance the incorporation of strontium, zinc and/or magnesium relative to calcium in the growing coral.

## Patentansprüche

1. Verfahren zur Herstellung eines Korallengerüsts zur Verwendung als Knochenersatzmaterial, wobei das Verfahren umfasst:
Züchten einer Koralle in einem Wachstumsmedium,
Entnehmen wenigstens eines Teils der Koralle aus dem Wachstumsmedium, und
Devitalisieren der dem Wachstumsmedium entnommenen Koralle,
wobei der pH-Wert des Wachstumsmediums auf einen pH-Wert von 8,3 oder höher eingestellt wird, und wobei das Wachstumsmedium eine Karbonathärte, dKH, besitzt, die auf weniger als 10 eingestellt ist.

2. Verfahren wie in Anspruch 1 beansprucht, wobei die dKH des Wachstumsmediums bei weniger als 10 gehalten wird und der pH-Wert des Wachstumsmediums bei einem pH-Wert von 8,3 bis 8,8 gehalten wird.

3. Verfahren wie in Anspruch 2 beansprucht, wobei der pH-Wert des Wachstumsmediums bei einem Soll-pH-Wert von 8,4 bis 8,6 gehalten wird.

4. Verfahren wie in einem der vorhergehenden Ansprüche beansprucht, wobei der pH-Wert des Wachstumsmediums durch Zugabe eines Gruppe-I- oder Gruppe-II-Metallhydroxids eingestellt wird.

5. Verfahren wie in einem der vorhergehenden Ansprüche beansprucht, wobei der pH-Wert des Wachstumsmediums durch Zugabe von Natriumhydroxid eingestellt wird.

6. Verfahren wie in Anspruch 1 beansprucht, wobei das Wachstumsmedium eine Karbonathärte, dKH, besitzt, die auf weniger als 8 eingestellt ist.

7. Verfahren wie in einem der vorhergehenden Ansprüche beansprucht, wobei das Wachstumsmedium eine Karbonathärte, dKH, besitzt, die auf 7 oder weniger eingestellt ist.

8. Verfahren wie in einem der vorhergehenden Ansprüche beansprucht, wobei das Wachstumsmedium eine Karbonathärte, dKH, besitzt, die auf 6,5 oder weniger eingestellt ist.

9. Verfahren wie in einem der vorhergehenden Ansprüche beansprucht, wobei das Wachstumsmedium eine Karbonathärte, dKH, besitzt, die auf weniger als 6 eingestellt ist.

10. Verfahren wie in einem der vorhergehenden Ansprüche beansprucht, umfassend das Hinzugeben von:
(i) Ammoniumhydroxid und/oder Alkalimetallhydroxid und
(ii) Hydrogencarbonat
zum Wachstumsmedium.

11. Verfahren wie in Anspruch 10 beansprucht, ferner umfassend das Messen des pH-Werts und der dKH des Wachstumsmediums und das Hinzugeben von (i) Gruppe-I-, Gruppe-II- oder Übergangsmetallhydroxid(en) und (ii) Hydrogencarbonat zum Wachstumsmedium, um den pH-Wert und die dKH innerhalb eines Sollbereichs zu halten.

12. Verfahren wie in einem der vorhergehenden Ansprüche beansprucht, ferner umfassend das Hinzugeben von Zink-, Magnesium- und/oder Strontiumchlorid zum Wachstumsmedium.

13. Verfahren wie in einem der vorhergehenden Ansprüche beansprucht, wobei Phosphat im Verhältnis zu Calcium zum Wachstumsmedium hinzugegeben wird, wodurch der Phosphatgehalt des resultierenden Knochenersatzmaterials eingestellt wird.

14. Verfahren wie in einem der vorhergehenden Ansprüche beansprucht, wobei die dKH unabhängig vom pH-Wert eingestellt wird.

15. Verfahren wie in einem der vorhergehenden Ansprüche beansprucht, wobei der Einbau von Strontium, Zink und/oder Magnesium in die Koralle durch Überwachen der Strontium-, Zink- und/oder Magnesiumkonzentration im Wachstumsmedium und Einstellen der Strontium-, Zink- und/oder Magnesiumkonzentration innerhalb von Sollgrenzen gesteuert wird, und gegebenenfalls wobei der pH-Wert des Wachstumsmediums eingestellt wird, um den Einbau von Strontium, Zink und/oder Magnesium relativ zu Calcium in der wachsenden Koralle zu fördern.

## Revendications

1. Procédé de fabrication d'un échafaudage corallien destiné à être utilisé comme substitut de greffon osseux, le procédé comprenant
la culture de corail dans un milieu de croissance,
l'enlèvement d'au moins une partie du corail du milieu de croissance, et la dévitalisation du corail enlevé du milieu de croissance,
dans lequel le pH du milieu de croissance est régulé à un pH de 8,3 ou supérieur, et dans lequel le milieu de croissance a une dureté carbonatée, dKH, [qui] est régulée à moins de 10.

2. Procédé selon la revendication 1, dans lequel la dKH du milieu de croissance est maintenue à moins de 10 et le pH du milieu de croissance est maintenu à un pH de 8,3 à 8,8.

3. Procédé selon la revendication 2, dans lequel le pH du milieu de croissance est maintenu à un pH ciblé de 8,4 à 8.6.

4. Procédé selon l'une quelconque des revendications précédentes, dans lequel le pH du milieu de croissance est régulé par l'ajout d'un hydroxyde de métal du groupe I ou du groupe II.

5. Procédé selon l'une quelconque des revendications précédentes, dans lequel le pH du milieu de croissance est régulé par l'ajout d'hydroxyde de sodium.

6. Procédé selon la revendication 1, dans lequel le milieu de croissance a une dureté carbonatée, dKH, régulée à moins de 8.

7. Procédé selon l'une quelconque des revendications précédentes, dans lequel le milieu de croissance a une dureté carbonatée, dKH, régulée à 7 ou moins.

8. Procédé selon l'une quelconque des revendications précédentes, dans lequel le milieu de croissance a une dureté carbonatée, dKH, régulée à 6.5 ou moins.

9. Procédé selon l'une quelconque des revendications précédentes, dans lequel le milieu de croissance a une dureté carbonatée, dKH, régulée à moins de 6.

10. Procédé selon l'une quelconque des revendications précédentes, qui comprend l'alimentation du milieu de croissance :
(i) en hydroxyde d'ammonium et/ou hydroxyde de métal alcalin, et
(ii) en bicarbonate

11. Procédé selon la revendication 10, qui comprend en outre la mesure du pH et de la dKH du milieu de croissance et l'alimentation en (i) groupe I, groupe II ou en hydroxide(s) de métaux de transition, et en (ii) bicarbonate dans le milieu de croissance pour maintenir le pH et la dKH dans une plage cible.

12. Procédé selon l'une quelconque des revendications précédentes, qui comprend en outre l'alimentation du milieu de croissance en chlorure de zinc, de magnésium et/ou de strontium.

13. Procédé selon l'une quelconque des revendications précédentes, dans lequel du phosphate est ajouté au milieu de croissance en proportion avec le calcium, régulant ainsi la teneur en phosphate du substitut de greffon osseux résultant.

14. Procédé selon l'une quelconque des revendications précédentes, dans lequel la dKH est régulée indépendamment du pH.

15. Procédé selon l'une quelconque des revendications précédentes, dans lequel l'incorporation de strontium, de zinc et/ou de magnésium dans le corail est régulée en surveillant la concentration de strontium, de zinc et/ou de magnésium dans le milieu de croissance et en ajustant la concentration de strontium, de zinc et/ou de magnésium dans des limites cibles, et éventuellement dans lequel le pH du milieu de croissance est ajusté pour augmenter l'incorporation de strontium, de zinc et/ou de magnésium par rapport au calcium dans le corail en croissance.
